# EUROPÄISCHE PATENTSCHRIFT

(11) **EP 1 178 782 B1**
(45) Veröffentlichungstag und Bekanntmachung des Hinweises auf die Patenterteilung: **09.08.2006**
(21) Anmeldenummer: 00931217.4
(22) Anmeldetag: 16.05.2000
(51) Int. Cl.: A61K 9/70, A61K 31/135

(54) **TRANSDERMALES THERAPEUTISCHES SYSTEM (TTS) TOLTERODIN ENTHALTEND**
TRANSDERMAL THERAPEUTIC SYSTEM (TTS) CONTAINING TOLTERODINE
SYSTEME THERAPEUTIQUE TRANSDERMIQUE (STT) CONTENANT DE LA TOLTERODINE

(30) Priorität: 18.05.1999 DE 19922662
(43) Veröffentlichungstag der Anmeldung: 13.02.2002
(73) Patentinhaber: SCHWARZ PHARMA AG, 40789 Monheim (DE)
(72) Erfinder: ARTH, Christoph, 40593 Düsseldorf (DE); MEESE, Claus, 40789 Monheim (DE); SCHACHT, Dietrich, Wilhelm, 50935 Köln (DE); WOLFF, Hans-Michael, 40789 Monheim (DE)
(74) Vertreter: Albrecht, Thomas
(86) Internationale Anmeldenummer: PCT/EP2000/004360
(87) Internationale Veröffentlichungsnummer: WO 2000/069421

(56) Entgegenhaltungen:
- WO-A-00/12069
- WO-A-00/12070
- WO-A-98/03067
- DATABASE WPI Section Ch, Week 199508 Derwent Publications Ltd., London, GB; Class A96, AN 1995-057300 XP002153780 & JP 06 336434 A (DAIICHI PHARM CO LTD), 6. Dezember 1994 (1994-12-06)

## Beschreibung

Die vorliegende Erfindung betrifft ein Transdermales Therapeutisches System (TTS) zur transcutanen Verabreichung von Tolterodin über mehrere Tage sowie Verfahren zu seiner Herstellung.

Die Bioverfügbarkeit von oral verabreichten Wirkstoffen ist oft unbefriedigend. Die intravenöse Verabreichung von Wirkstoffen ist für die Patienten häufig unangenehm und unbefriedigend. Die hepatische Metabolisierung vieler Wirkstoffe kann bei der ersten Leberpassage zu unerwünschten Konzentrationsverhältnissen, toxischen Nebenprodukten und zur Verminderung der Wirkung oder gar zum Wirkverlust führen. Gegenüber oraler Ver-abreichung besitzt die transdermale Gabe von Wirkstoffen verschiedene Vorteile. Die Wirkstoffzufuhr läßt sich über einen längeren Zeitraum besser steuern, wodurch hohe Blutspiegelschwankungen vermieden werden. Zudem kann die erforderliche therapeu-tisch wirksame Dosis meist deutlich verringert werden. Außerdem wird ein Pflaster vom Patienten oft mehr bevorzugt als täglich einmal oder mehrfach einzunehmende Tabletten.

In der Vergangenheit wurde zur Überwindung der vorgenannten Nachteile der nichttransdermalen Gabe von Wirkstoffen durch eine Vielzahl von Transdermalen Therapeutischen Systemen (TTS) mit unterschiedlichem Aufbau für verschiedene Wirkstoffe zur Therapie unterschiedlicher Erkrankungen Rechnung getragen.

So beschreiben die nachfolgend genannten technischen Dokumente für eine breite Vielfalt systemisch oder lokal reagierender Wirkstoffe deren parenterale Verabreichung entweder auf Basis dosis-kontrollierender oder allgemein freisetzender Systeme. Beispielhaft sind dies U.S. P. (U.S.-Patentnummem) 3,598,122 A; 3,598,123 A; 3,731,683 A; 3,797,494 A; 4,031,894 A; 4,201,211 A; 4,286,592 A; 4,314,557 A; 4,379,454 A; 4,435,180 A; 4,559,222 A; 4,568,343 A; 4,573,995 A, 4,588,580 A; 4,645,502 A; 4,702,282 A; 4,788,062 A; 4,816,258 A; 4,849,226 A; 4,908,027 A; 4,943,435 A und 5,004,610 A.

In den späten sechziger Jahren dieses Jahrhunderts war ursprünglich theoretisch angenommen worden, daß jeder Wirkstoff mit kurzer Halbwertszeit aber hoher Wirksamkeit und guter Hautdurchgängigkeit für eine sichere und effektive Verabreichung mittels eines TTS geeignet sei. Diese anfänglichen Erwartungen hinsichtlich der Möglichkeiten der transdermalen Verabreichung von Wirkstoffen mittels TTS konnten jedoch nicht erfüllt werden. Dies findet seine Begründung hauptsächlich darin, daß die Haut von Natur aus mit einer unüberschaubaren Vielfalt von Eigenschaften ausgestattet ist, um ihre Funktion als intakte Barriere gegenüber dem Eindringen von nicht-körpereigenen Substanzen in den Körper aufrecht zu erhalten. (Siehe hierzu: Transdermal Drug Delivery: Problems and Possibilities, B.M. Knepp et al., CRC Critical Review and Therapeutic Drug Carrier Systems, Vol. 4, Issue 1 (1987)).

Daher steht die transdermale Verabreichung nur für diejenigen wenigen Wirkstoffe zur Verfügung, die eine geeignete Kombination von vielen günstigen Charakteristika aufweisen. Für einen bestimmten Wirkstoff sind diese geforderten Charakteristika, die die sichere und effektive transdermale Verabreichung gewährleisten sollen, jedoch nicht vorhersagbar.

Die an einen für die transdermale Verabreichung geeigneten Wirkstoff zu stellenden Anforderungen sind:
- Hautdurchgängigkeit,
- keine Beeinträchtigung des Klebevermögens des Pflasters durch den Wirkstoff,
- Vermeidung von Hautirritationen,
- Vermeidung von allergischen Reaktionen,
- günstige pharmakokinetische Eigenschaften,
- günstige pharmakodynamische Eigenschaften,
- ein relativ weites therapeutisches Fenster,
- Metabolismuseigenschaften, die konsistent mit der therapeutischen Anwendung bei kontinuierlicher Gabe sind.

Unzweifelhaft ist die vorgenannte Liste der Anforderungen nicht erschöpfend. Damit ein Wirkstoff für die transdermale Verabreichung zur Verfügung stehen kann, ist die "richtige" Kombination all dieser Anforderungen wünschenswert.

Das für die Wirkstoffe vorgenannte gilt in gleicher Weise für die den jeweiligen Wirkstoff enthaltende TTS-Zusammensetzung und deren konstruktiven Aufbau.

Üblicherweise handelt es sich bei den Transdermalen Therapeutischen Systemen (TTS) um Pflaster, die mit einer undurchlässigen Deckschicht, einer abziehbaren Schutzschicht und einer wirkstoffhaltigen Matrix oder einem wirkstoffhaltigen Reservoir mit semipermeabler Membran ausgestattet sind. Im ersten Fall werden sie als Matrixpflaster, im zweiten Fall als Membransystem bezeichnet.

Für die Deckschicht werden üblicherweise Polyester, Polypropylen, Polyethylen, Polyurethan etc. verwendet, die auch metallisiert oder pigmentiert sein können. Für die abziehbare Schutzschicht kommen u.a. Polyester, Polypropylen oder auch Papier mit Silikon- und/oder Polyethylenbeschichtung in Betracht. Auch Fluoropolymere finden Anwendung.

Für die pharmazeutisch bzw. medizinisch üblichen wirkstoffhaltigen Matrices werden vielfach Stoffe auf Basis von Polyacrylat, Silikon, Polyisobutylen, Butylkautschuk, Styrol/Butadien-Copolymerisat oder Styrol/Isopren-Copolymerisat verwendet.

Die in Membransystemen verwendeten Membranen können mikroporös oder semipermeabel sein und werden üblicherweise auf Basis eines inerten Polymeren, insbesondere Polypropylen, Polyvinylacetat oder Silikon gebildet.

Während die wirkstoffhaltigen Matrixzusammensetzungen selbstklebend sein können, ergeben sich aber auch in Abhängigkeit vom eingesetzten Wirkstoff wirkstoffhaltige Matrices, die nicht selbstklebend sind, so daß als Folge hiervon das Pflaster oder TTS konstruktiv mit einem Overtape versehen werden muß.

Zur Sicherstellung der erforderlichen Fluxrate des Wirkstoffes sind häufig Hautpenetrationsenhancer wie aliphatische, cycloaliphatische und/oder aromatisch-aliphatische Alkohole, jeweils ein- oder mehrwertig und jeweils mit bis zu 8 C-Atomen umfassend, ein Alkohol-/Wasser-Gemisch, ein gesättigter und/oder ungesättigter Fettalkohol mit jeweils 8 bis 18 Kohlenstoffatomen, eine gesättigte und/oder ungesättigte Fettsäure mit jeweils 8 bis 18 Kohlenstoffatomen und/oder deren Ester sowie Vitamine als Zusatz erforderlich.

Weiterhin werden häufig Stabilisatoren, wie Polyvinylpyrrolidon, α-Tocopherolsuccinat, Propylgallat, Methionin, Cystein und/oder Cystein-hydrochlorid, der wirkstoffhaltigen Matrix zugesetzt.

Wie die vorgenannte Aufstellung zeigt, sind zahlreiche TTS-Konstruktionen und hierfür verwendete Materialien bekannt. Allerdings sind viele interagierende Erfordemisse zu berücksichtigen, wenn ein Medikament in Form eines TTS einem medizinischen Bedürfnis genügen soll.

Die folgenden Problemstellungen sind bei der Entwicklung von wirkstoffhaltigen TTS zu berücksichtigen:
1. Die Hautpermeabilität für den Wirkstoff ist zu niedrig, um die therapeutisch notwendige Penetrationsrate zu erzielen und/oder die Verzögerungszeit ("lag-time") bis zum Erreichen der therapeutisch erforderlichen Plasmaspiegel ist zu lang, mit der Folge, daß hautpenetrationsbeschleunigende Zusätze verabreicht werden müssen.
2. Die wirkstoffbeladene und ggf. zusätzlich mit Hautpenetrationsenhancem beladene Polymermatrix ist bei längerer Lagerung physikalisch nicht stabil. Insbesondere kann eine Wirkstoffrekristallisation auftreten, die zu einer nicht kontrollierbaren Abnahme der Wirkstofffreisetzungskapazität des TTS führt.
3. Eine hohe Beladung des polymeren Trägerstoffes mit Wirkstoff und/oder Hautpenetrationsenhancem erschwert bei selbstklebenden Polymerfilmen die Einstellung optimaler Hafteigenschaften des transdermalen Systems.
4. Die Wirkstoffresorptionsrate sinkt bei Anwendungen über mehrere Tage in nicht akzeptabler Weise ab, so daß zusätzliche Steuerschichten und/oder -komponenten erforderlich sind.
5. Ferner ist aus der Literatur bekannt, daß die zur Penetrationsförderung durch die Haut häufig eingesetzten Fettsäuresester mehrwertiger Alkohole schwankende Qualität und unreine Verschnittmittel aufweisen. Dies führt zu schlecht reproduzierbaren Penetrationsteigerungen (Burkoth et al. 1996, DE 196 22 902 A1).

Die beschriebenen Probleme bedingen daher eine Vielzahl von Ausführungsformen Transdermaler Therapeutischer Systeme, die sich im Stand der Technik auf diesem Gebiet widerspiegeln.

DE 196 53 606 A1 beschreibt ein Haft- und Bindemittel für TTS aus jeweils definierten Massenanteilen der Komponenten a) (Meth)acrylatpolymer, das quartäre Ammoniumgruppen aufweisen kann, b) eine organische Di- oder Tricarbonsäure und c) einen Weichmacher, der ein Citronensäuretriester sein kann.

Wie die vorgenannte Aufstellung zeigt, sind viele Pflasterkonstruktionen und hierfür verwendete Materialien bekannt. Gleichwohl besteht bis heute für viele in Transdermalen Therapeutischen Systemen verarbeitete Wirkstoffe ein großer Bedarf, TTS zur Verfügung zu stellen, die eine therapeutisch geforderte Wirkstoffabgabe ermöglichen, ohne dabei konstruktiv aufwendig zu sein und in der Gesamtschau ihrer Bestandteile eine optimale Beziehung darstellen. Dies gilt auch für den Wirkstoff Tolterodin, wenn er transcutan verabreicht werden soll.

Tolterodin ist die generische Bezeichnung (INN) für das R-Isomer von N,N-Diisopropyl-3-(2-hydroxy-5-methylphenyl)-3-phenylpropylamin (IUPAC-Bezeichnung (+)-(R)-2-{α[2-(Diisopropylamino)ethyl]benzyl}4-methylphenol). Als Tolterodin wird im folgenden N,N-Diisopropyl-3-(2-hydroxy-5-ethylphenyl)-3-phenylpropylamin bezeichnet. Soweit die einzelnen Isomere, also das R- oder S-Isomer hiervon oder die razemische Mischung von R- und S-Isomer betroffen sind, sind diese als R-, S- bzw. R,S-Tolterodin bezeichnet.

Therapeutisch verwendet wird Tolterodin zur Behandlung der instabilen Hamblase verbunden mit den Symptomen imperativer Harndrang. Pollakisurie und Drang-Inkontinenz. Die empfohlene Dosis ist zweimal täglich 2 mg Tolterodin die oral verabreicht werden.

Nach oraler Applikation wird Tolterodin bei der ersten Leberpassage in stark unterschiedlichem Ausmaß metabolisiert. So beträgt die absolute Bioverfügbarkeit von Tolterodin bei langsamen Metabolisierem 65 %, bei schnellen Metabolisierem aber nur 17 %. Da auch der entstehende 5-Hydroxymethyl-Metabolit pharmakologisch aktiv ist, haben die geringeren Tolterodin-Blutspiegel bei den schnellen Metabolisierern keinen Wirkungsverlust im gleichen Umfang zur Folge. Gleichwohl ist es wünschenswert, derartige interindividuelle Schwankungen zu vermeiden und sich hieraus ergebende Wirkungsunterschiede grundsätzlich zu vermeiden. Weiterhin ergeben sich unterschiedliche Plasmaspiegel, wenn die Verabreichung von Tolterodin mit oder ohne Aufnahme von Nahrung erfolgt. Diese Probleme sind durch die transdermale Verabreichung von Tolterodin grundsätzlich vermeidbar, da der Wirkstoff hierbei unter Umgehung des Magen-DarmTraktes und der ersten Leberpassage direkt dem Blutkreislauf zugeführt wird. Durch transdermale Verabreichung können bei oraler Verabreichung sich ergebende Plasmaschwankungen mit hohen Konzentrationsspitzen vermieden werden, die zu unerwünschten Nebenwirkungen wie Mundtrockenheit, Dyspepsie, Erbrechen, Akkomodationsstörungen und Verwirrung führen können. Ebenso können unter die Wirkungsschwelle abfallende Wirkstoffspiegel und unbeabsichtigtes Hamlassen rund um die Uhr vermieden werden. Darüber hinaus ergibt sich durch die Umgehung der ersten Leberpassage eine deutlich geringere Belastung der Leber mit dem Wirkstoff, was insbesondere für Patienten mit vorgeschädigter Leber wie z.B. bei Patienten mit Leberzirrhose wünschenswert ist.

WO 98/03067 A1 lehrt die Verwendung von S-Tolterodin zur Behandlung von Blasenentleerungsstörungen einschließlich der Inkontinenz. Für die Verabreichung des Wirkstoffes wird u.a. auch die transdermale Applikation vorgeschlagen. Eine technische Lehre zur Ausführung der transdermalen Applikation oder ein hierauf gerichtetes Ausführungsbeispiel sind jedoch nicht enthalten.

Transdermale Therapeutische Systeme zur Verabreichung von Tolterodin sind im Stand der Technik nach Art. 54(2) EPÜ nicht beschrieben.

Die WO 00/12069 und die WO 00/12070, die Dokumente nach Artikel 54(3) EPÜ darstellen, betreffen tolterodinhaltige Formulierungen mit verzögerter Freisetzung bzw. für die transdermale Verabreichung.

Es ist daher Aufgabe der vorliegenden Erfindung, ein TTS für Tolterodin zur Verfügung zu stellen. Das TTS sollte konstruktiv einfach aufgebaut, gut hautverträglich und über längere Lagerungs- und Applikationsdauer physikalisch und chemisch stabil sein, gute Hafteigenschaften aufweisen und pro Flächeneinheit möglichst viel Wirkstoff an und durch die Haut freisetzen.

Diese Aufgabe wurde gelöst, indem ein Transdermales Therapeutisches System (TTS) zur transcutanen Verabreichung von Tolterodin zur Verfügung gestellt wird, das eine selbstklebende, schichtförmige Matrixmasse enthält, die ein ammoniogruppenhaltiges (Meth)acrylatcopolymer, mindestens.einen Weichmacher und bis zu 25 Gew.-% Tolterodin enthält, wobei das TTS Tween 80 enthält. Überraschenderweise wird Tolterodin hieraus in so hoher Geschwindigkeit an und durch die Haut freigesetzt, wie dies für andere Wirkstoffe nur in Verbindung mit Hautdurchdringungsverstärkem bekannt ist. Somit kann die therapeutisch erforderliche Dosierung mit TTS mit kleiner Freisetzungsfläche erfolgen, ohne daß ein erhöhtes Risiko von Hautreizungen durch Hautdurchdringungsverstärkem in Kauf genommen werden muß.

Im Sinne der Erfindung werden die Begriffe "mehrere Tage" und "feste Lösung" wie folgt verstanden:
a) "mehrere Tage": die TTS können zur therapeutischen Anwendung von 1 bis zu 7, vorzugsweise 1 bis 4 Tagen auf die Haut appliziert werden.
b) "feste Lösung": der pharmazeutische Wirkstoff liegt in der TTS-Matrix molekulardispers verteilt vor.

Nach einer weiteren erfindungsgemäßen Ausführung kann das vorbeschriebene TTS zusätzlich mit Ausnahme der Freisetzungsfläche seiner Tolterodin-haltigen Matrix auf der Haut von einem größeren, jedoch wirkstofffreien Hautpflaster zur Fixierung an der Applikationsstelle (Overtape) umgeben sein.

Dieser Aufbau bedingt den Vorteil, daß den verschiedenen Hauttypen und Klimazonen Rechnung getragen werden kann. Weiterhin können einerseits die Ko-/Adhäsionseigenschaften des TTS, andererseits die Wirkstofflöslichkeit, Wirkstofflösungsgeschwindigkeit und das Freisetzungsverhalten weitgehend getrennt voneinander optimiert werden.

Bevorzugt enthält die wirkstoffhaltige Matrix R-Tolterodin oder R,S-Tolterodin.

Nach einer weiteren Ausführungsform der Erfindung ist in der Matrixmasse deuteriertes Tolterodin als Wirkstoff enthalten. Deuteriertes Tolterodin liegt vor, wenn in Tolterodin ein oder mehrere Wasserstoffatome durch dessen isotop Deuterium ausgetauscht ist. Grundsätzlich kann jedes der in Tolterodin enthaltenen Wasserstoffatome durch Deuterium ersetzt sein. Bevorzugt enthält der Methylsubstituent des Aromaten und/oder dieser Aromate selbst mindestens ein Deuteriumatom.

Beispielhaft hierfür genannt ist 2-(3-Diisopropylamino-1-phenylpropyl)-4-[²H₃]methylphenol.

Überraschenderweise wurde gefunden, daß die Hautpenetrationsgeschwindigkeit von deuteriertem Tolterodin gegenüber nicht deuteriertem Tolterodin, das ohnehin bereits eine sehr hohe Hautdurchdringungsgeschwindigkeit aufweist, nochmals deutlich erhöht ist.

Nach einer Weiterbildung enthält die Matrixmasse vorzugsweise 10 - 20 Gew.-% Tolterodin.

Schließlich kann die Tolterodin-haltige Matrixmasse eine feste Lösung sein.

Die Bildung einer festen Lösung des Tolterodins in dem ammoniogruppenhaltigen (Meth)acrylatpolymeren war nicht vorherzusehen und ist umso überraschender, als viele Wirkstoffe in Polymeren keine festen Lösungen (mit molekulardisperser Verteilung) bilden, sondern sich in Form fester Teilchen in das jeweilige Polymer einlagern, die elektronenmikroskopisch zu erkennen sind. Im Gegensatz zu festen Lösungen zeigen kristalline Wirkstoffe auch ein Debye-Scherrer-Diagramm.

Nach einer weiteren Ausführungsform der Erfindung enthält die Tolterodin-haltige Matrixmasse mindestens einen Zitronensäuretriester. Bevorzugt enthält der Zitronensäuretriester kurzkettige Alkansäuren. Hierbei kommen insbesondere Methansäure, Ethansäure, n-Propansäure, i-Propansäure, n-Butansäure, sek. Butansäure und tert. Butansäure in Betracht.

Nach einer bevorzugten Ausführungsform ist in der Tolterodin-haltigen Matrixmasse Zitronensäure(n)-butylester, Zitronensäureethylester oder eine Mischung hieraus enthalten.

Aufgrund der erfindungsgemäßen Zusammensetzung und des konstruktiven Aufbaus des TTS ist es überraschend, daß trotz hoher Wirkstoffkonzentrationen an Tolterodin in der Polymermatrix eine ausreichende physikalische Stabilität des Systems bei Langzeitlagerung gewährleistet ist.

Für das als wirkstoffhaltige Polymermatrix verwendete Polymer war nicht zu erwarten, daß unmittelbar nach dem Aufkleben des TTS ein inniger Kontakt zwischen Wirkstoffmatrix und Haut hergestellt ist, der von der Qualität ist, daß ein über mehrere Tage selbständig klebendes TTS resultiert, das sowohl den therapeutischen als auch den gewerblichen, insbesondere den betriebswirtschaftlichen Erfordemissen, genügt.

Der Patientencompliance wird damit hervorragend Rechnung getragen.

Wenn man die Ausführungsform mit einem wirkstofffreien Hautpflaster/Overtape wählt, sind nur sehr kleinflächige Hautpflaster mit einem nur wenige mm Breite aufweisenden Kleberand erforderlich.

Dies ist sowohl wirtschaftlich als auch bezüglich der Patientencompliance von Vorteil.

Nach einer weiteren Ausführungsform der Erfindung weist die Trägerfolie des TTS matrixseitig eine Metalldampf- oder Oxidbeschichtung auf.

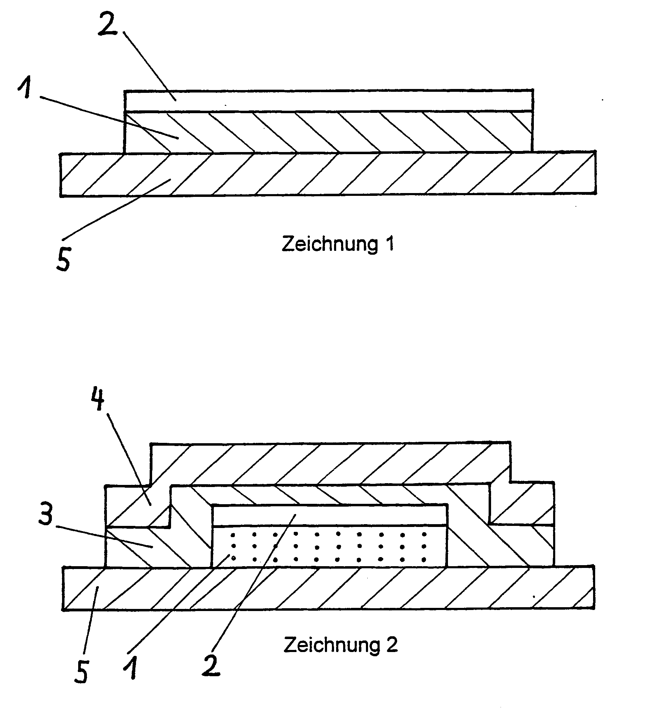

Der Aufbau der erfindungsgemäßen TTS ist in Zeichnungen 1 und 2 dargestellt.

Zeichnung 1 zeigt die Ausführungsform ohne Overtape, bestehend aus wirkstoffhaltiger Polymermatrix (1), ablösbarer Schutzfolie (5) und Deckfolie (2).
Zeichnung 2 zeigt die Ausführungsform mit Overtape. Zusätzlich zu den in der in Zeichnung 1 dargestellten Ausführungsform enthaltenen Schichten ist ein Overtape bestehend aus Trägerfolie (4) und Adhäsivfilm (3) enthalten.

Das erfindungsgemäße TTS kann nach dem sog. "Lösungsmittel-basierten Verfahren" hergestellt werden. Hierzu werden Polymer, Wirkstoff und die sonstigen Inhaltsstoffe in einem gemeinsamen Lösungsmittel gelöst und die erhaltene Lösung in dünner Schicht auf einem Träger verteilt. Der beschichtete Träger wird getrocknet, um das in der Polymermatrix enthaltene Lösungsmittel zu entfernen, mit einer weiteren Folie abgedeckt und anschließend in Stücke gewünschter Größe vereinzelt.

Alternativ kann das TTS auch nach dem sogenannten "Hot-melt-Verfahren" hergestellt werden. Hierzu wird das Polymer aufgeschmolzen, mit dem Wirkstoff sowie den übrigen Hilfsstoffen vermischt, die erhaltene Mischung in dünner Schicht auf eine(n) Träger(folie) (= ablösbare Schutzfolie) verteilt und erkalten lassen. Anschließend wird mit einer weiteren Folie abgedeckt (Deckfolie) und in Stücke gewünschter Größe vereinzelt.

Bevorzugt erfolgt die Herstellung der Tolterodin-haltigen Matrixmasse durch Schmelzextrusion, wobei der wirksame Bestandteil in eine Schmelze aus Polymer und Weichmacher kontinuierlich als Festsubstanz zudosiert wird und die erhaltene wirkstoffhaltige Polymerschmelze sofort nach erfolgter Zudosierung des Wirkstoffs kontinuierlich auf eine ablösbare Schutzschicht in einer Dicke von 0,02 bis 0,5 mm beschichtet wird und das erhaltene 2-Schichtlaminat auf der anderen Matrixseite mit einer Deckschicht versehen wird. Herstellung der wirkstoffhaltigen Matrixmasse und deren weitere Verarbeitung erfolgen vorteilhaft in einem kontinuierüchen und kosteneinsparenden Arbeitsgang mit kurzen Prozeßzeiten. Die thermische Belastung der wirkstoffhaltigen Polymermasse wird auf ein Minimum reduziert, so daß Zersetzungsreaktionen ausgeschlossen sind.

Die Erfindung wird anhand folgender Beispiele erläutert:

### Beispiel 1

2,52 g Eudragit RS 100, (= Poly (ethylacrylat, methylmethacrylat, trimethylammonioethylmethacrylatchlorid) mit einem molaren Verhältnis der Monomereinheiten von 1:2:0,1)
1,16 g Tributylcitrat und
0,65 g R-Tolterodin werden unter Zugabe von
8,00 g Essigsäureethylester in einem Becherglas durch Rühren gelöst.

Die erhaltene Polymerlösung wird mit einem Streichrakel auf eine mit Aluminium bedampfte und beidseitig silikonisierte ca. 100 µm dicke, wieder ablösbare Polyesterfolie (= Trägerfolie) ausgestrichen und 30 Minuten bei 45°C im Umlufttrockenschrank getrocknet, so daß ein Tolterodin-haltiger Polymerfilm mit einem Flächengewicht von 110 g/m² resultiert. Dieser wird anschließend mit einer ca. 19 µm dicken Polyesterfolie (= Deckfolie) abgedeckt. Aus dem so erhaltenen 3-schichtig aufgebauten Laminat, bestehend aus ablösbarer Schutzfolie, wirkstoffhaltigem Polymerfilm und Deckfolie werden 5 cm² große Transdermale Systeme (TTS) ausgestanzt.

### Tolterodin-Fluxmessungen in vitro

### Fluxmessungen durch Mäusehaut

Ein TTS mit einer ausgestanzten Fläche von 2,55 cm² wird in einer horizontalen Diffusionszelle auf die Homschichtseite der Bauch- und Rückenhaut haarloser Mäuse fixiert. Unmittelbar anschließend wird die Akzeptorkammer der Zelle mit auf 32°C vortemperierter Phosphat-Pufferlösung (0,066 molar) pH 6,2 luftblasenfrei befüllt und das Freisetzungsmedium auf 32 ± 0,5°C thermostatisiert.

Zu den Probeentnahmezeiten (nach 3, 6, 24, 30, 48, 54 und 72 Stunden) wird das Freisetzungsmedium gegen frisches, auf 32 ± 0,5°C thermostatisiertes Medium ausgetauscht.

### Fluxmessungen durch Humanhaut

Die Prüfung erfolgte in einer Durchflußzelle nach der von Tiemessen (Harry L.G.M. Thiemessen et al., Acta Pharm. Technol. 34 (1988), 99-101) beschriebenen Methode an frisch präparierter, ca. 200 µm dicker Humanhaut, die zur Akzeptorseite hin auf einer Silikonmembran aufliegt (Akzeptormedium: Phosphatpufferlösung (0,066 molar) pH 6,2; thermostatisiert auf 32 ± 0,5°C).

Die Probeentnahmen erfolgten nach 3, 6, 9, 12, 15, 18, 21, 24, 27, 30, 33, 36, 39, 42, 45, 48, 51, 54, 57, 60, 63, 66, 69 und 72 Stunden.

Der Gehalt an R-Tolterodin Base im Freisetzungs- bzw. Akzeptormedium bei den Humanhautpermeationsuntersuchungen wird mittels Hochleistungsflüssigkeitschromatographie unter den nachfolgend aufgeführten Bedingungen bestimmt. Stationäre Phase: C₈-Umkehrphase, 3,9 x 150 mm, 5 µm; Säulentemperatur: Raumtemperatur; Eluent: 700 Volumenteile Natriumdihydrogenphosphatpuffer (0,05 mol) pH 3,0, 300 Volumenanteile Acetonitril; Detektion: UV bei 220 nm; Flußrate: 1,2 ml/min; Injektionsvolumen: 50 µl bei 15°C.

Die Ergebnisse der Untersuchungen für Beispiel 1 sind in Tabelle 1 dargestellt.

**Tabelle 1:**

| R-Tolterodin Base Fluxraten durch exzidierte Hautpräparation (Beispiel 1, Ch.-B. INZ 006) | | | | |
|---|---|---|---|---|
| | Gehalt an R-Tolterodin-Base [Gew.-%] | Mittlerer kumulativer Flux [µg/cm²] nach | | |
| | | 24 h | 48 h | 72 h |
| Mäusehaut (n = 4) | 15,0 | 524,2 | 849,0 | 1036,4 |
| Humanhaut (n = 3) | 15,0 | 130,5 | 460,1 | 731,0 |

### Beispiel 2

9,71 g Eudragit RS 100, (=Poly (ethylacrylat, methylmethacrylat, trimethylammonioethylmethacrylatchlorid) mit einem molaren Verhältnis der Monomereinheiten von 1:2:0,1)
4,76 g Tributylcitrat und
2,50 g (R,S)-Tolterodin werden unter Zugabe von
32,00 g Essigsäureethylester in einem Becherglas durch Rühren gelöst.

Die erhaltene Polymerlösung wird mit einem Streichrakel auf eine mit Aluminium bedampfte und beidseitig silikonisierte ca. 100 µm dicke, wieder ablösbare Polyesterfolie (= Trägerfolie) ausgestrichen und 30 Minuten bei 45°C im Umlufttrockenschrank getrocknet, so daß ein Tolterodin-haltiger Polymerfilm mit einem Flächengewicht von 125 g/m² resultiert. Dieser wird anschließend mit einer ca. 19 µm dicken Polyesterfolie (= Deckfolie) abgedeckt. Aus dem so erhaltenen 3-schichtig aufgebauten Laminat, bestehend aus wieder ablösbarer Schutzfolie, wirkstoffhaltigem Polymerfilm und Deckfolie werden 5 cm² große TTS ausgestanzt.

### Tolterodin-Fluxmessungen in vitro

### Fluxmessungen durch Mäusehaut

Ein TTS mit einer ausgestanzten Fläche von 2,55 cm² wird in einer horizontalen Diffusionszelle auf die Homschichtseite der Bauch- und Rückenhaut haarloser Mäuse fixiert. Unmittelbar anschließend wird die Akzeptorkammer der Zelle mit auf 32°C vortemperierter Phosphat-Pufferlösung (0,066 molar) pH 6,2 luftblasenfrei befüllt und das Freisetzungsmedium auf 32 ± 0,5°C thermostatisiert.

Zu den Probeentnahmezeiten (nach 3, 6, 24, 30, 48, 54 und 72 Stunden) wird das Freisetzungsmedium gegen frisches, auf 32 ± 0,5°C thermostatisiertes Medium ausgetauscht.

### Fluxmessungen durch Humanhaut

Die Prüfung erfolgte in einer Durchflußzelle nach der von Tiemessen (Harry L.G.M. Thiemessen et al., Acta Pharm. Technol. 34 (1988), 99-101) beschriebenen Methode an frisch präparierter, ca. 200 µm dicker Humanhaut, die zur Akzeptorseite hin auf einer Silikonmembran aufliegt (Akzeptormedium: Phosphatpufferlösung (0,066 molar) pH 6,2; thermostatisiert auf 32 ± 0,5°C).

Die Probeentnahmen erfolgten nach 3, 6, 9, 12, 15, 18, 21, 24, 27, 30, 33, 36, 39, 42, 45, 48, 51, 54, 57, 60, 63, 66, 69 und 72 Stunden.

Der Gehalt an (R,S)-Tolterodin Base im Freisetzungs- bzw. Akzeptormedium bei den Humanhautpermeationsuntersuchungen wird mittels Hochleistungsflüssigkeitschromatographie unter den nachfolgend aufgeführten Bedingungen bestimmt. Stationäre Phase: C₈-Umkehrphase, 3,9 x 150 mm, 5 µm; Säulentemperatur: Raumtemperatur; Eluent: 700 Volumenteile Natriumdihydrogenphosphatpuffer (0,05 mol) pH 3,0, 300 Volumenanteile Acetonitril; Detektion: UV bei 220 nm; Flußrate: 1,2 ml/min.; Injektionsvolumen: 50 µl bei 15°C.

Die Ergebnisse der Untersuchungen für Beispiel 2 sind in Tabelle 2 dargestellt.

**Tabelle 2:**

| (R,S)-Tolterodin Base Fluxraten durch exzidierte Hautpräparation (Beispiel 2, Ch.-B. INZ 007) | | | | |
|---|---|---|---|---|
| | Gehalt an (R,S) Tolterodin-Base [Gew.-%] | Mittlerer kumulativer Flux [µg/cm²] nach | | |
| | | 24 h | 48 h | 72 h |
| Mäusehaut (n = 4) | 15,0 | 648,8 | 1110,4 | 1302,0 |
| Humanhaut (n = 4) | 15,0 | 208,4 | 718,9 | 1219,4 |

### Beispiel 3

9,71 g Eudragit RS 100, (=Poly (ethylacrylat, methylmethacrylat, trimethylammonioethylmethacrylatchlorid) mit einem molaren Verhältnis der Monomereinheiten von 1:2:0,1)
4,76 g Tributylcitrat und
2,50 g R-(+)-2-(3-Diisopropylamino-1-phenyl-propyl)-4-[²H₃]methylphenol(R-(D₃)-Tolterodin) werden unter Zugabe von
32,00 g Essigsäureethylester in einem Becherglas durch Rühren gelöst.

Die erhaltene Polymerlösung wird mit einem Streichrakel auf eine mit Aluminium bedampfte und beidseitig silikonisierte ca. 100 µm dicke, wieder ablösbare Polyesterfolie (= Trägerfolie) ausgestrichen und 30 Minuten bei 45°C im Umlufttrockenschrank getrocknet, so daß ein Tolterodin-haltiger Polymerfilm mit einem Flächengewicht von 125 g/m² resultiert. Dieser wird anschließend mit einer ca. 19 µm dicken Polyesterfolie (= Deckfolie) abgedeckt. Aus dem so erhaltenen 3-schichtig aufgebauten Laminat, bestehend aus wieder ablösbarer Schutzfolie, wirkstoffhaltigem Polymerfilm und Deckfolie werden 5 cm² große TTS ausgestanzt.

### Tolterodin-Fluxmessungen in vitro

### Fluxmessungen durch Mäusehaut

Ein TTS mit einer ausgestanzten Fläche von 2,55 cm² wird in einer horizontalen Diffusionszelle auf die Homschichtseite der Bauch- und Rückenhaut haarloser Mäuse fixiert. Unmittelbar anschließend wird die Akzeptorkammer der Zelle mit auf 32°C vortemperierter Phosphat-Pufferiösung (0,066 molar) pH 6,2 luftblasenfrei befüllt und das Freisetzungsmedium auf 32 ± 0,5°C thermostatisiert.

Zu den Probeentnahmezeiten (nach 3, 6, 24, 30, 48, 54 und 72 Stunden) wird das Freisetzungsmedium gegen frisches, auf 32 ± 0,5°C thermostatisiertes Medium ausgetauscht.

Der Gehalt an R-(D₃)-Tolterodin Base im Freisetzungs- bzw. Akzeptormedium bei den Mäusehautpermeationsuntersuchungen wird mittels Hochleistungsflüssigkeitschromatographie unter den nachfolgend aufgeführten Bedingungen bestimmt. Stationäre Phase: C₈-Umkehrphase, 3,9 x 150 mm, 5 µm; Säulentemperatur. Raumtemperatur, Eluent: 700 Volumenteile Natriumdihydrogenphosphatpuffer (0,05 mol) pH 3,0, 300 Volumenanteile Acetonitril; Detektion: UV bei 220 nm; Flußrate: 1,2 ml/min.; Injektionsvolumen: 50 µl bei 15°C.

### Fluxmessungen durch Humanhaut

Die Prüfung erfolgte in einer Durchflußzelle nach der von Tiemessen (Harry L.G.M. Thiemessen et al., Acta Pharm. Technol. 34 (1988), 99-101) beschriebenen Methode an frisch präparierter, ca. 200 µm dicker Humanhaut, die zur Akzeptorseite hin auf einer Silikonmembran aufliegt (Akzeptormedium: Phosphatpufferlösung (0,066 molar) pH 6,2; thermostatisiert auf 32 ± 0,5°C).

Die Probeentnahmen erfolgten nach 3, 6, 9, 12, 15, 18, 21, 24, 27, 30, 33, 36, 39, 42, 45, 48, 51, 57, 60, 63, 66, 69 und 72 Stunden.

Der Gehalt an R-(D₃)-Tolterodin Base im Freisetzungs- bzw. Akzeptormedium bei den Humanhautpermeationsuntersuchungen wird mittels Hochleistungsflüssigkeitschromatographie unter den nachfolgend aufgeführten Bedingungen bestimmt. Stationäre Phase: C₈-Umkehrphase, 3,9 x 150 mm, 5µm; Säulentemperatur: Raumtemperatur, Eluent: 700 Volumenteile Natriumdihydrogenphosphatpuffer (0,05 mol) pH 3,0, 300 Volumenanteile Acetonitril, Detektion: UV bei 220 nm; Flußrate: 1,2 ml/min; Injektionsvolumen: 50 µl bei 15°C.

Die Ergebnisse der Untersuchungen sind für Beispiel 3 in Tabelle 3 dargestellt.

**Tabelle 3:**

| R-(D₃)-Tolterodin Base Fluxraten durch exzidierte Hautpräparation (Beispiel 3, Ch.-B. INZ 013) | | | | |
|---|---|---|---|---|
| | Gehalt an R-(D₃)-Tolterodin-Base [Gew.-%] | Mittlerer kumulativer Flux [µg/cm²] nach | | |
| | | 24 h | 48 h | 72 h |
| Mäusehaut (n = 4) | 15,0 | 872,0 | 1388,4 | 1737,4 |
| Humanhaut (n = 3) | 15,0 | 165,9 | 490,8 | 784,2 |

## Patentansprüche

1. Transdermales Therapeutisches System (TTS) zur transcutanen Verabreichung von Tolterodin über mehrere Tage, **dadurch gekennzeichnet, dass** das TTS eine selbstklebende, schichtförmige Matrixmasse aufweist, die ein ammoniogruppenhaltiges (Meth)acrylatcopolymer, mindestens einen Weichmacher und bis zu 25 Gew.-% Tolterodin enthält, wobei das TTS kein Tween 80 enthält.

2. TTS nach Anspruch 1, **dadurch gekennzeichnet, dass** es eine Fixierungshilfe für das TTS auf der Haut aufweist und dieses, mit Ausnahme seiner Freisetzungsfläche, an der Applikationsstelle von einem größeren wirkstofffreien Pflaster zur Fixierung an der Haut umgeben ist.

3. TTS nach Ansprüchen 1-2, **dadurch gekennzeichnet, dass** die wirkstoffhaltige Matrixmasse (R,S)-Tolterodin oder R-Tolterodin enthält.

4. TTS nach Ansprüchen 1-3, **dadurch gekennzeichnet, dass** die wirkstoffhaltige Matrixmasse deuteriertes Tolterodin enthält.

5. TTS nach Ansprüchen 1-4, **dadurch gekennzeichnet, dass** die Tolterodinhaltige Matrixmasse eine feste Lösung ist.

6. TTS nach Ansprüchen 1-5, **dadurch gekennzeichnet, dass** die Tolterodinhaltige Matrixmasse mindestens einen Zitronensäuretriester als Weichmacher enthält.

7. TTS nach Anspruch 6, **dadurch gekennzeichnet, dass** als Weichmacher Zitronensäuretributylester alleine oder in Mischung mit Zitronensäuretriethylester enthalten ist.

8. TTS nach Ansprüchen 1-7, **dadurch gekennzeichnet, dass** die Trägerfolie matrixseitig eine Metalldampf- oder Oxidbeschichtung aufweist.

## Claims

1. A transdermal therapeutic system (TTS) for transcutaneously administering tolterodine over a period of several days, **characterised in that** the TTS comprises a self-adhesive, layer-shaped matrix material containing an ammonio group-containing (meth)acrylate copolymer, at least one softener and up to 25 wt. % of tolterodine, wherein the TTS contains no Tween 80.

2. The TTS according to claim 1, **characterised in that** it comprises a means of fixing the TTS on the skin and which, with the exception of its release area, at its application site, is surrounded by a larger plaster free from active ingredients for fixing to the skin.

3. The TTS according to claims 1-2, **characterised in that** the matrix material containing an active ingredient contains (R,S)-tolterodine or (R)-tolterodine.

4. The TTS according to claims 1-3, **characterised in that** the matrix material containing an active ingredient contains deuterated tolterodine.

5. The TTS according to claims 1-4, **characterised in that** the matrix material containing tolterodine is a solid solution.

6. The TTS according to claims 1-5, **characterised in that** the matrix material containing tolterodine contains at least one triester of citric acid as the softener.

7. The TTS according to claim 6, **characterised in that** tributyl ester of citric acid, alone or mixed with triethyl ester of citric acid, is contained as the softener.

8. The TTS according to claims 1-7, **characterised in that** the carrier foil comprises a metal vapour or oxide coating on the side of the matrix material.

## Revendications

1. Système thérapeutique transdermique (STT) pour l'administration transcutanée de toltérodine pendant plusieurs jours, **caractérisé en ce que** le STT comprend une masse formant matrice, autocollante et stratiforme, qui contient un copolymère de (méth)acrylate contenant des groupes ammonio, au moins un plastifiant et jusqu'à 25 % en poids de toltérodine, le STT ne contenant pas de Tween 80.

2. STT selon la revendication 1, **caractérisé en ce qu'**il comprend un auxiliaire de fixation du STT sur la peau, et ce STT, à l'exception de sa zone de libération, est sur le site d'application entouré d'un emplâtre plus grand, et exempt de principe actif, pour fixation à la peau.

3. STT selon les revendications 1-2, **caractérisé en ce que** la masse formant matrice et contenant le principe actif contient de la (R,S)-toltérodine ou de la R-toltérodine.

4. STT selon les revendications 1 à 3, **caractérisé en ce que** la masse formant matrice et contenant le principe actif contient de la toltérodine deutériée.

5. STT selon les revendications 1 à 4, **caractérisé en ce que** la masse formant matrice et contenant la toltérodine est une solution solide.

6. STT selon les revendications 1 à 5, **caractérisé en ce que** la masse formant matrice et contenant la toltérodine contient au moins un triester de l'acide citrique en tant que plastifiant.

7. STT selon la revendication 6, **caractérisé en ce qu'**il contient en tant que plastifiant l'ester butylique de l'acide citrique, seul ou en mélange avec de l'ester triéthylique de l'acide citrique.

8. STT selon les revendications 1 à 7, **caractérisé en ce que** la feuille support présente, côté matrice, un revêtement métrallisé, ou d'oxyde.
